# EUROPÄISCHE PATENTSCHRIFT

(11) **EP 0 632 165 B1**
(45) Veröffentlichungstag und Bekanntmachung des Hinweises auf die Patenterteilung: **13.05.1998**
(21) Anmeldenummer: 94112628.6
(22) Anmeldetag: 13.07.1992
(51) Int. Cl.: E01C 19/28, E02D 3/02, G01P 1/10

(54) **Verfahren zum Feststellen und Anzeigen der beim Arbeiten mit einem Bodenverdichtungsgerät erreichten Bodendichte**
Process for determining and indicating the degree of ground compacting attained during work with a ground compactor
Procédé de détermination et d'indication de la densité du sol obtenu par un engin de compactage du sol

(30) Priorität: 20.07.1991 DE 4124193
(43) Veröffentlichungstag der Anmeldung: 04.01.1995
(62) Teilanmeldung aus: 92915538.0
(73) Patentinhaber: WACKER-WERKE GmbH & Co. KG, D-85084 Reichertshofen (DE)
(72) Erfinder:
(74) Vertreter: Müller, Frithjof E., Dipl.-Ing.

(56) Entgegenhaltungen:
- EP-A- 0 092 484
- EP-A- 0 281 683
- PATENT ABSTRACTS OF JAPAN vol. 15, no. 279 (M-1136) 16. Juli 1991 & JP-A-03 096 637 (MAZDA MOTOR CORP.) 22. April 1991
- DATABASE WPI Section EI, Week 9138, Derwent Publications Ltd., London, GB; Class S02, AN 91-280001 & SU-A-1 603 213 (SELKHOZMASHAVTOMATT) 30. Oktober 1990

## Beschreibung

Die Erfindung bezieht sich auf ein Verfahren gemäß dem Oberbegriff des Patentanspruchs 1.

Bodenverdichtungsgeräte werden in der Regel mit konstanter Stellung des Liefergradhebels, d. h. des die Energiezufuhr steuernden Hebels, betrieben.

Es ist bekannt (z. B. aus dem Dokument EP-A-0 281 683) zur Echtzeit-Feststellung und -Anzeige des beim Arbeiten mit einem Bodenverdichtungsgerät erreichten Grades der Bodendichte einen die Belastung des Motors wiederspiegelnden Motorparameter zu überwachen und in ein entsprechendes elektrisches Signal umzusetzen. Geeignete Betriebsparameter sind in diesem Zusammenhang bei Verbrennungskraftmaschinen z. B. das von der Motorwelle übertragene Drehmoment und die Ladung des Motors.

Die direkte Erfassung des jeweiligen Betriebsparameters mit guter Genauigkeit insbesondere in dem bei Bodenverdichtungsarbeiten besonders interessanten Endbereich des anzustrebenden Verdichtungsgrades ist jedoch bisher schwierig gewesen und erforderte einen hohen baulichen Aufwand.

Es ist bekannt, Verdichtungsgeräte mit Motoren anzutreiben, die über eine Überdrehzahl-Schutzeinrichtung verfügen.

Aufgabe der Erfindung ist es, ein Verfahren gemäß dem einleitenden Teil des Patentanspruchs 1 zu schaffen, das mit einem verhältnismäßig kleinen baulichen Aufwand durchführbar ist und sehr genaue Ergebnisse insbesondere im Endbereich der anzustrebenden Bodenverdichtung, also dort, wo die Entscheidung zu treffen ist, mit der Verdichtungsarbeit aufzuhören, liefert.

Die vorstehende Aufgabe wird durch die im Patentanspruch 1 genannten Merkmale gelöst.

Das Ausmaß der Kraftstoffzufuhr zum Verbrennungsmotor ist kein direkter Betriebsparameter des Motors, doch spiegelt bei Verwendung eines Motors mit Überdrehzahl-Schutzregelkreis wegen der Tätigkeit des letzteren das Ausmaß der Minderung der Kraftstoffzufuhr dennoch den augenblicklichen Lastfall des Motors insbesondere zum Ende der Verdichtung eines gerade in Bearbeitung befindlichen Bodens hin sehr genau wieder und läßt sich auch auf besonders einfache Weise feststellen und in ein entsprechendes elektrisches Signal umsetzen.

In diesem Zusammenhang ist es besonders günstig, gemäß Anspruch 2 einen Überdrehzahl-Schutzregelkreis einzusetzen, der die Kraftstoffzufuhr mittels einer mechanischen Regeleinrichtung steuert, und die Stellung dieser mechanischen Regeleinrichtung mittels eines Sensors zu erfassen und zur Bildung der Anzeigegröße heranzuziehen.

Der Verbrennungsmotor kann als solcher von üblicher Bauart sein.

Bei dem von dem Motor angetriebenen Bodenverdichtungsgerät kann es sich um eine Verdichtungsplatte, einen Stampfer, eine Walze oder dgl. von bekannter Konstruktion handeln.

## Patentansprüche

1. Verfahren zum Feststellen und Anzeigen des Grades der von einem Bodenverdichtungsgerät während des Betriebes augenblicklich jeweils erreichten Bodendichte, **dadurch gekennzeichnet,** daß
a) das Verdichtungsgerät mittels eines Verbrennungsmotors angetrieben wird, der von einem Überdrehzahl-Schutzregelkreis, welcher bei Überschreiten einer vorbestimmten maximalen Drehzahl die Kraftstoffzufuhr zeitweise sperrt, gegen Überschreiten des vorbestimmten Drehzahlmaximalwertes gesichert ist, und
b) die Zeitdauer und/oder die Häufigkeit der Sperrungen der Kraftstoffzufuhr in einem Zeitintervall festgestellt und zur Bildung einer Anzeigegröße für den augenblicklich jeweils erreichten Bodenverdichtungsgrad herangezogen wird.

2. Verfahren nach Anspruch 1, unter Verwendung eines Überdrehzahl-Schutzregelkreises, der die Kraftstoffzufuhr mittels einer mechanischen Regeleinrichtung steuert, **dadurch gekennzeichnet**, daß die Stellung der mechanischen Regeleinrichtung mittels eines Sensors erfaßt und zur Bildung der Anzeigegröße herangezogen wird.

## Claims

1. Process for determining and indicating the degree of ground compacting attained by a ground compactor at any moment during operation, characterised in that
a) the compactor is driven by means of a combustion engine which is protected against exceeding a predetermined engine speed maximum value by an overspeed protection control circuit which shuts off the supply of fuel for a time in the case of the predetermined maximum engine speed being exceeded, and
b) the duration and/or frequency of the fuel supply shut-offs is determined in a time interval and is used to form an indication variable for the momentarily achieved degree of ground compacting.

2. Process according to claim 1, using an overspeed protection control circuit which controls the supply of fuel by means of a mechanical control device, characterised in that the position of the mechanical control device is detected by means of a sensor and is used to form the indication variable.

## Revendications

1. Procédé destiné à constater et à indiquer le degré de la densité du sol atteinte à l'instant par un dispositif de compactage du sol pendant le fonctionnement, caractérisé en ce que
a) le dispositif de compactage est entraîné au moyen d'un moteur à combustion interne, qui est protégé contre un dépassement de la valeur de vitesse de rotation maximale prédéterminée, par un circuit de régulation et de protection contre les survitesses, qui ferme provisoirement l'arrivée de carburant en cas de dépassement d'une vitesse de rotation maximale prédéterminée et
b) la durée et/ou la fréquence des fermetures d'arrivée de carburant pendant un intervalle de temps est déterminée et utilisée pour former une grandeur d'affichage du degré de compactage du sol, obtenu à l'instant.

2. Procédé selon la revendication 1, avec utilisation d'un circuit de régulation et de protection contre les survitesses, qui commande l'arrivée de carburant au moyen d'un dispositif de régulation mécanique, caractérisé en ce que la position du dispositif de régulation mécanique est détectée au moyen d'un capteur et utilisée pour former la grandeur d'affichage.
